# EUROPEAN PATENT APPLICATION

(11) **EP 4 707 267 A1**
(43) Date of publication of application: **11.03.2026**
(21) Application number: 25796442.9
(22) Date of filing: 18.07.2025
(51) Int. Cl.: C07C 17/42, C07B 63/04, C07C 21/18

(54) **COMPOSITION COMPRISING HEXAFLUOROPROPENE**

(30) Priority: 26.07.2024 JP 2024121040; 26.07.2024 JP 2024121044; 26.07.2024 JP 2024121052
(71) Applicant: DAIKIN INDUSTRIES, LTD., Osaka-Shi, Osaka 530-0001 (JP)
(72) Inventor: ETO, Yusuke, Osaka-shi, Osaka 530-0001 (JP); SUGIYAMA, Akinari, Osaka-shi, Osaka 530-0001 (JP); KUROKI, Yoshichika, Osaka-shi, Osaka 530-0001 (JP); NAKAMURA, Shingo, Osaka-shi, Osaka 530-0001 (JP)
(74) Representative: Hoffmann Eitle
(86) International application number: PCT/JP2025/025776
(87) International publication number: WO 2026/023578

(57) **Abstract**

The present disclosure aims to provide a novel hexafluoropropene-containing composition. A hexafluoropropene-containing composition is provided.

## Description

### Technical Field

The present disclosure relates to a hexafluoropropene-containing composition.

### Background Art

PLT 1 discloses a method of etching a silicon-based material such as a silicon oxide film and/or a low-dielectric constant film containing silicon by using hexafluoropropene.

### Citation List

### Patent Literature

PTL 1: WO0221586-A1

### Summary of Invention

### Technical Problem

The present disclosure aims to provide a novel hexafluoropropene-containing composition.

### Solution to Problem

The present disclosure encompasses the following features.

### Item 1.

A hexafluoropropene-containing composition comprising:
(1) hexafluoropropene;
(2) pentafluoropropene; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

### Item 2.

The composition according to Item 1,
wherein, based on the total amount of the composition,
the hexafluoropropene (1) is present at 95 vol% to 99.99999 vol%, and
the pentafluoropropene (2) is present at 1 ppm by volume to 1,000 ppm by volume.

### Item 3.

A method for storing a hexafluoropropene-containing composition,
the composition comprising:
(1) hexafluoropropene;
(2) pentafluoropropene; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

### Item 4.

The method according to Item 3,
wherein, based on the total amount of the composition,
the hexafluoropropene (1) is present at 95 vol% to 99.99999 vol%, and
the pentafluoropropene (2) is present at 1 ppm by volume to 1,000 ppm by volume.

### Item 5.

A method for suppressing a decrease in the purity of hexafluoropropene in a hexafluoropropene-containing composition, the composition comprising:
(1) hexafluoropropene;
(2) pentafluoropropene; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

### Item 6.

The method according to Item 5,
wherein, based on the total amount of the composition,
the hexafluoropropene (1) is present at 95 vol% to 99.99999 vol%, and
the pentafluoropropene (2) is present at 1 ppm by volume to 1,000 ppm by volume.

The present disclosure discloses a method for stably transporting a composition containing hexafluoropropene (HFP) and pentafluoropropene. According to the present disclosure, the oxygen concentration, hydrogen fluoride concentration, or hydrogen chloride concentration in the HFP-containing composition is adjusted, which makes it possible to suppress the decomposition of HFP or pentafluoropropene and suppress a decrease in the purity of HFP or pentafluoropropene during transportation of the HFP-containing composition in cylinders or the like, whereby the storage stability of the HFP-containing composition can be maintained.

The HFP-containing composition of the present disclosure contains pentafluoropropene. Thus, the composition has improved storage stability and can be stably transported by suppressing the decomposition of HFP or pentafluoropropene even under harsh marine transportation conditions in containers or the like (e.g., conditions such as temperature increase during transportation).

### Advantageous Effects of Invention

The present disclosure can provide a novel hexafluoropropene-containing composition.

### Description of Embodiments

In the present specification, the terms "include," "contain," and variations thereof express concepts that encompass all of "comprise," "consist essentially of," and "consist of." In the present specification, when a numerical range is expressed as "A to B," the expression means A or more and B or less. In the present specification, when expressions such as "part(s)" and "%" are used, these mean parts by mass, parts by weight, mass%, or wt%.

### [1] Hexafluoropropene-containing composition

The hexafluoropropene (HFP)-containing composition of the present disclosure comprises:
(1) hexafluoropropene (HFP);
(2) pentafluoropropene; and
(3) at least one component selected from the group consisting of oxygen (O₂), hydrogen fluoride (HF), and hydrogen chloride (HCl),
wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

Preferably, in the HFP-containing composition of the present disclosure,
based on the total amount of the composition,
the HFP (1) is present at 95 vol% to 99.99999 vol%, and
the pentafluoropropene (2) is present at 1 ppm by volume to 1,000 ppm by volume.

The present disclosure discloses a method for stably transporting a composition containing HFP and pentafluoropropene. According to the present disclosure, the oxygen concentration, hydrogen fluoride concentration, or hydrogen chloride concentration in an HFP-containing composition is adjusted, which makes it possible to suppress the decomposition of HFP or pentafluoropropene and suppress a decrease in the purity of HFP or pentafluoropropene during transportation of the HFP-containing composition in cylinders or the like, whereby the storage stability of the HFP-containing composition can be maintained.

The HFP-containing composition of the present disclosure contains pentafluoropropene. Thus, the composition has improved storage stability and can be stably transported by suppressing the decomposition of HFP or pentafluoropropene even under harsh marine transportation conditions in containers or the like (e.g., conditions such as temperature increase during transportation).

### (1) Hexafluoropropene

The HFP-containing composition of the present disclosure contains (1) hexafluoropropene (CF₃-CF=CF₂, also known as hexafluoropropylene, HFP) as a first component.

The HFP-containing composition of the present disclosure contains the first component "(1) HFP" as a main component. For improved storage stability and stable transportation by suppressing the decomposition of HFP or pentafluoropropene even under harsh marine transportation conditions in containers or the like (e.g., conditions such as temperature increase during transportation), the amount of HFP in the HFP-containing composition based on the total amount (gas volume) of the HFP-containing composition is preferably 95 vol% to 99.99999 vol%, more preferably 95 vol% to 99.999 vol% or 96 vol% to 99.99 vol%, even more preferably 97 vol% to 99.95 vol%, and particularly preferably 98 vol% to 99.9 vol%.

### (2) Pentafluoropropene

The HFP-containing composition of the present disclosure contains (2) pentafluoropropene (C₃HF₅) as a second component.

The HFP-containing composition of the present disclosure contains pentafluoropropene (C₃HF₅) as a second component. Thus, the composition has improved storage stability and can be stably transported by suppressing the decomposition of HFP or its dimer and/or trimer even under harsh marine transportation conditions in containers or the like (e.g., conditions such as temperature increase during transportation).

There is no limitation on pentafluoropropene as the second component, and any isomer thereof can be used. A preferred pentafluoropropene for use is 1,1,2,3,3-pentafluoropropene (F₂C=CF-CHF₂), 1,1,3,3,3-pentafluoropropene (F₂C=CH-CF₃), (E/Z)-1,2,3,3,3-pentafluoro-1-propene (HFC=CF-CF₃), or the like.

In the HFP-containing composition, one pentafluoropropene (including isomers) may be used alone or two or more thereof may be used (blended) as a mixture.

For improved storage stability and stable transportation by suppressing the decomposition of HFP or pentafluoropropene even under harsh marine transportation conditions in containers or the like (e.g., conditions such as temperature increase during transportation), the amount of pentafluoropropene in the HFP-containing composition based on the total amount (gas volume) is preferably 1 ppm by volume (ppm volume) to 1,000 ppm by volume (ppm volume), more preferably 10 ppm by volume to 500 ppm by volume, even more preferably 10 ppm by volume to 300 ppm by volume, and particularly preferably 50 ppm by volume to 300 ppm by volume.

### (3) Oxygen, hydrogen fluoride, and hydrogen chloride

The HFP-containing composition of the present disclosure contains, as a third component, (3) at least one component selected from the group consisting of oxygen (O₂), hydrogen fluoride (HF), and hydrogen chloride (HCl).

### (a) Oxygen (O₂)

The HFP-containing composition of the present disclosure contains (3) oxygen (O₂) as a third component.

The amount of oxygen (O₂) in the HFP-containing composition based on the total amount (gas volume) of the HFP-containing composition is 0.05 ppm by volume or more and 10 ppm by volume or less. The O₂ concentration in the HFP-containing composition is adjusted within the above range, which makes it possible to suppress the decomposition of HFP or pentafluoropropene and suppress a decrease in the purity of HFP or pentafluoropropene in the HFP-containing composition during transportation of the HFP-containing composition in cylinders or the like, whereby the storage stability of the HFP-containing composition can be well maintained. The HFP-containing composition can be stably transported by suppressing the decomposition of HFP or pentafluoropropene even under harsh marine transportation conditions in containers or the like (e.g., conditions such as temperature increase during transportation).

The amount of oxygen (O₂) in the HFP-containing composition based on the total amount (gas volume) of the HFP-containing composition is preferably 0.1 ppm by volume (ppm volume) to 10 ppm by volume (ppm volume), and more preferably 0.1 ppm by volume to 5 ppm by volume.

### (b) Hydrogen fluoride (HF)

The HFP-containing composition of the present disclosure contains (3) hydrogen fluoride (HF) as a third component.

The amount of hydrogen fluoride (HF) in the HFP-containing composition based on the total amount (gas volume) of the HFP-containing composition is 0.05 ppm by volume or more and 5 ppm by volume or less. The HF concentration in the HFP-containing composition is adjusted within the above range, which makes it possible to suppress the decomposition of HFP or pentafluoropropene and suppress a decrease in the purity of HFP or pentafluoropropene during transportation of the HFP-containing composition in cylinders or the like, whereby the storage stability of the HFP-containing composition can be well maintained. The HFP-containing composition can be stably transported by suppressing the decomposition of HFP or pentafluoropropene even under harsh marine transportation conditions in containers or the like (e.g., conditions such as temperature increase during transportation).

The amount of hydrogen fluoride (HF) in the HFP-containing composition based on the total amount (gas volume) of the HFP-containing composition is preferably 0.1 ppm by volume (ppm volume) to 5 ppm by volume (ppm volume), more preferably 0.1 ppm by volume to 3 ppm by volume, and even more preferably 0.1 ppm by volume to 1 ppm by volume.

### (c) Hydrogen chloride (HCl)

The HFP-containing composition of the present disclosure contains (3) hydrogen chloride (HCl) as a third component.

The amount of hydrogen chloride (HCl) in the HFP-containing composition based on the total amount (gas volume) of the HFP-containing composition is 0.05 ppm by volume or more and 5 ppm by volume or less. The HCl concentration in the HFP-containing composition is adjusted within the above range, which makes it possible to suppress the decomposition of HFP or pentafluoropropene and suppress a decrease in the purity of HFP or pentafluoropropene during transportation of the HFP-containing composition in cylinders or the like, whereby the storage stability of the HFP-containing composition can be well maintained. The HFP-containing composition can be stably transported by suppressing the decomposition of HFP or pentafluoropropene even under harsh marine transportation conditions in containers or the like (e.g., conditions such as temperature increase during transportation).

The amount of hydrogen chloride (HCl) in the HFP-containing composition based on the total amount (gas volume) of the HFP-containing composition is preferably 0.1 ppm by volume (ppm volume) to 5 ppm by volume (ppm volume), more preferably 0.1 ppm by volume to 3 ppm by volume, and even more preferably 0.1 ppm by volume to 1 ppm by volume.

### [2] Method for storing hexafluoropropene-containing composition

The present disclosure encompasses a method for storing a hexafluoropropene (HFP)-containing composition and a method for suppressing a decrease in the purity of HFP in an HFP-containing composition.

In the method for storing an HFP-containing composition or the method for suppressing a decrease in the purity of HFP, the HFP-containing composition comprises:
(1) HFP;
(2) pentafluoropropene; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

Preferably, in the HFP-containing composition,
based on the total amount of the composition,
the HFP (1) is present at 95 vol% to 99.99999 vol%, and the pentafluoropropene (2) is present at 1 ppm by volume to 1,000 ppm by volume.

The details of the HFP-containing composition are as described in section [1] Hexafluoropropene-containing

### composition.

The present disclosure discloses a method for stably transporting a composition containing HFP and pentafluoropropene. According to the present disclosure, the oxygen (O₂) concentration, hydrogen fluoride (HF) concentration, or hydrogen chloride (HCl) concentration in the HFP-containing composition is adjusted, which makes it possible to suppress the decomposition of HFP or pentafluoropropene and suppress a decrease in the purity of HFP or pentafluoropropene in the HFP-containing composition during transportation of the HFP-containing composition in cylinders or the like, whereby the storage stability of the HFP-containing composition can be well maintained.

The HFP-containing composition of the present disclosure contains pentafluoropropene and suppresses the generation of an HFP-derived decomposed product or a pentafluoropropene-derived decomposed product. Thus, the composition has improved storage stability and can be stably transported by suppressing the decomposition of HFP or pentafluoropropene even under harsh marine transportation conditions in containers or the like (e.g., conditions such as temperature increase during transportation).

### [3] Application of hexafluoropropene-containing composition

The present disclosure encompasses a method for etching a semiconductor manufacturing substrate using, as an etching gas, the hexafluoropropene (HFP)-containing composition of the present disclosure, which is supplied as a plasma-generating gas.

A silicon oxide film (SiO₂ film) or a silicon-based material having a silicon nitride film (SiN film) formed thereon can be etched using a gas plasma of the HFP-containing composition (etching gas). The silicon-based material to be etched is preferably a semiconductor manufacturing substrate.

When hole etching is performed in a silicon oxide film (SiO₂ film) or a silicon-based material having a silicon nitride film (SiN film) formed thereon using a gas plasma generated from the HFP-containing composition, good etching can be achieved in which the processed hole shape is well maintained. The conditions for the etching method (in particular, the dry etching method) are the same as those for the conventional method.

### Examples

The present invention is specifically described below with reference to Examples and Comparative Examples, which do not limit the present invention.

### [1-1] Storage stability of hexafluoropropene-containing composition

### Example 1-1

Hexafluoropropene: HFP
Pentafluoropropene
Oxygen: O₂

According to a conventional method, HFP was purified, the pentafluoropropene concentration was adjusted to predetermined levels (200 ppm by volume, 100 ppm by volume, and 10 ppm by volume), and the O₂ concentration was adjusted to predetermined levels (100 ppm by volume, 10 ppm by volume, 5 ppm by volume, and 1 ppm by volume), whereby HFP-containing compositions were prepared.

The pentafluoropropene concentration (ppm by volume) in each HFP-containing composition was analyzed by gas chromatography (GC analysis).

The O₂ concentration (ppm by volume) in each HFP-containing composition was analyzed by GC.

The HFP-containing compositions were stored at a constant temperature (20°C or 50°C). After fixed periods (30 days, 90 days, and 180 days), the gas phase was extracted, and a hexafluoropropene (HFP)-derived decomposed product was analyzed by GC analysis (ppm by volume).

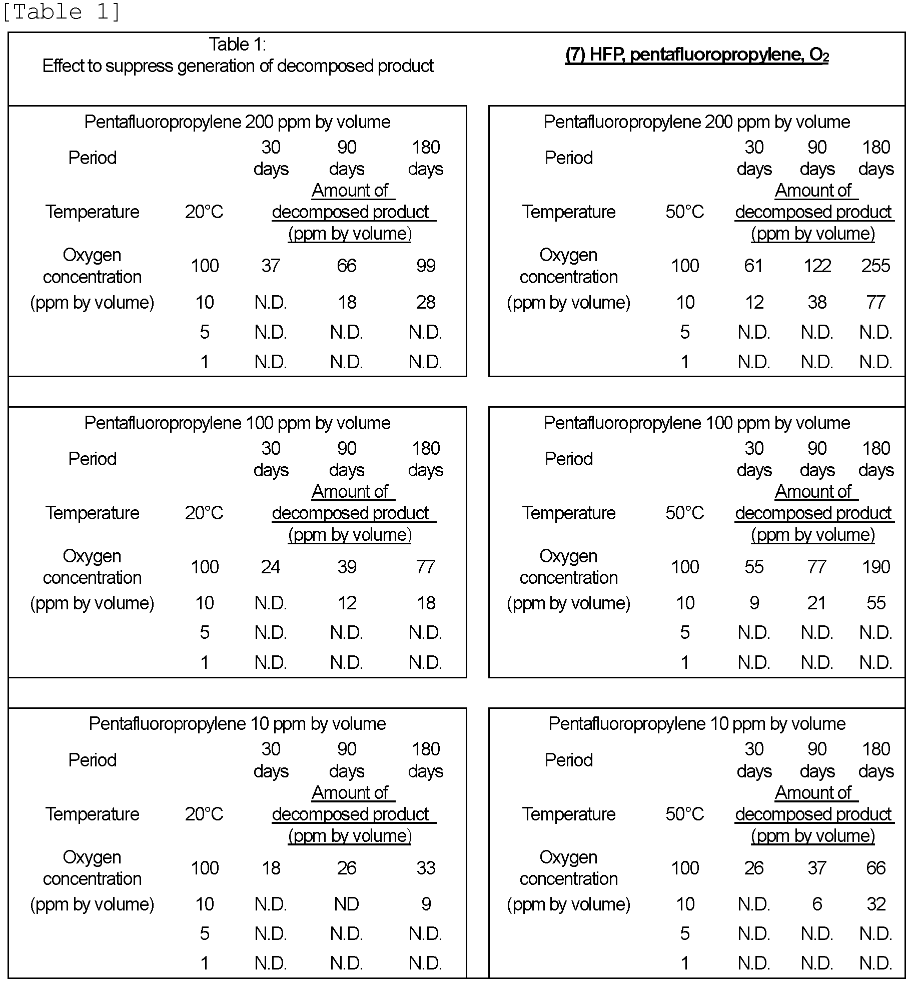

The compositions containing HFP and pentafluoropropene (200 ppm by volume, 100 ppm by volume, and 10 ppm by volume) of the Example in which the O₂ concentration was adjusted (10 ppm by volume or less) successfully suppressed the generation of an HFP-derived decomposed product or a pentafluoropropene-derived decomposed product even after the fixed periods (30 days, 90 days, and 180 days) of storage at a constant temperature (20°C or 50°C) .

### [1-2] Study of lower limit amount of oxygen (O₂) in composition

### Example 1-2

According to a conventional method, a composition containing HFP and pentafluoropropene (5 ppm by volume) was prepared, and the O₂ concentration was adjusted to predetermined levels (0.1 ppm by volume, 0.01 ppm by volume), whereby compositions containing O₂ and pentafluoropropene were prepared.

The O₂ concentration (ppm by volume) in each composition containing HFP and pentafluoropropene (5 ppm by volume) was analyzed by GC.

The compositions containing HFP and pentafluoropropene (5 ppm by volume) were stored at a constant temperature (20°C, 50°C). After fixed periods (30 days, 90 days, and 180 days), the gas phase was extracted and analyzed by GC (ppm by volume).

**[Table 2]**

| Table 2: Study of lower limit amount of oxygen | | | | |
|---|---|---|---|---|
| O₂ concentration (ppm by volume) | | Decomposed product (ppm by volume) | | |
| 20°C | | 30 days | 90 days | 180 days |
| Example 1-2-1 | 0.1 | N.D. | N.D. | N.D. |
| Comparative Example 1-1-1 | 0.01 | 0.02 | 0.03 | 0.05 |
| | | | | |

| O₂ concentration (ppm by volume) | | Decomposed product (ppm by volume) | | |
|---|---|---|---|---|
| 50°C | | 30 days | 90 days | 180 days |
| Example 1-2-2 | 0.1 | N.D. | N.D. | N.D. |
| Comparative Example 1-1-2 | 0.01 | 0.03 | 0.04 | 0.05 |

In the composition in which the O₂ concentration was 0.01 ppm by volume, no decomposed product was observed. In the composition in which the O₂ concentration was 0.01 ppm by volume, a decomposed product was detected.

### [2-1] Storage stability of hexafluoropropene-containing

### composition

### Example 2-1

Hexafluoropropene: HFP
Pentafluoropropene
Hydrogen fluoride: HF

According to a conventional method, HFP was purified, the pentafluoropropene concentration was adjusted to predetermined levels (200 ppm by volume, 100 ppm by volume, and 10 ppm by volume), and the HF concentration was adjusted to predetermined levels (10 ppm by volume, 5 ppm by volume, 1 ppm by volume, and 0.1 ppm by volume), whereby HFP-containing compositions were prepared.

The pentafluoropropene concentration (ppm by volume) in each HFP-containing composition was analyzed by gas chromatography (GC analysis).

The HF concentration (ppm by volume) in each HFP-containing composition was analyzed by a Fourier transform infrared spectrophotometer (FT-IR) to measure the initial concentration.

The HFP-containing compositions were stored at a constant temperature (20°C or 50°C). After fixed periods (30 days, 90 days, and 180 days), the gas phase was extracted, and a hexafluoropropene (HFP)-derived decomposed product was analyzed by GC analysis (ppm by volume).

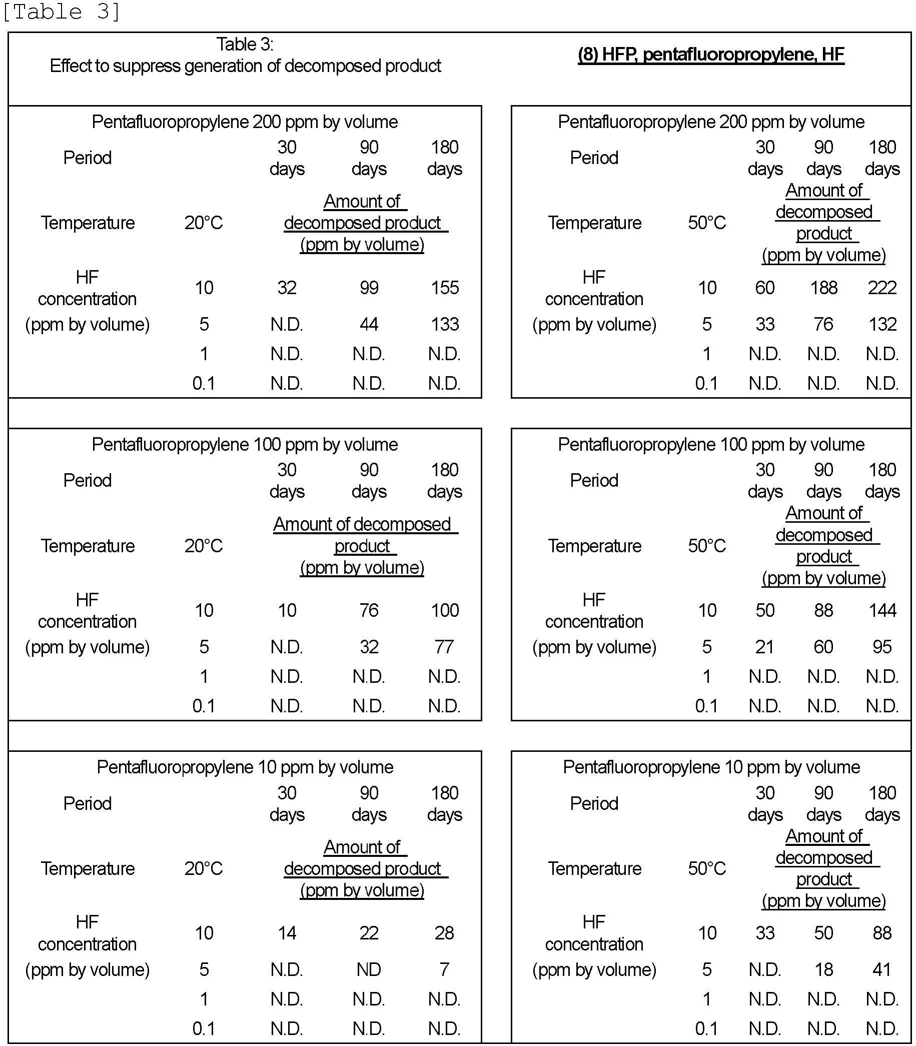

The compositions containing HFP and pentafluoropropene (200 ppm by volume, 100 ppm by volume, and 10 ppm by volume) of the Example in which the HF concentration was adjusted (5 ppm by volume or less) successfully suppressed the generation of an HFP-derived decomposed product or a pentafluoropropene-derived decomposed product even after the fixed periods (30 days, 90 days, and 180 days) of storage at a constant temperature (20°C or 50°C) .

### [2-2] Study of lower limit amount of hydrogen fluoride (HF) in composition

### Example 2-2

According to a conventional method, a composition containing HFP and pentafluoropropene (5 ppm by volume) was prepared, and the O₂ concentration was adjusted to predetermined levels (0.1 ppm by volume, 0.01 ppm by volume), whereby compositions containing HF and pentafluoropropene were prepared.

The HF concentration (ppm by volume) in each composition containing HFP and pentafluoropropene (5 ppm by volume) was analyzed by GC.

The compositions containing HFP and pentafluoropropene (5 ppm by volume) were stored at a constant temperature (20°C, 50°C). After fixed periods (30 days, 90 days, and 180 days), the gas phase was extracted and analyzed by GC (ppm by volume).

**[Table 4]**

| Table 4: Study of lower limit amount of hydrogen fluoride | | | | |
|---|---|---|---|---|
| HF concentration (ppm by volume) | | Decomposed product (ppm by volume) | | |
| 20°C | | 30 days | 90 days | 180 days |
| Example 2-2-1 | 0.1 | N.D. | N.D. | N.D. |
| Comparative Example 2-1-1 | 0.01 | 0.2 | 0.3 | 0.5 |
| | | | | |

| HF concentration (ppm by volume) | | Decomposed product (ppm by volume) | | |
|---|---|---|---|---|
| 50°C | | 30 days | 90 days | 180 days |
| Example 2-2-2 | 0.1 | N.D. | N.D. | N.D. |
| Comparative Example 2-1-2 | 0.01 | 0.3 | 0.5 | 0.6 |

In the composition in which the HF concentration was 0.1 ppm by volume, no decomposed product was observed. In the composition in which the HF concentration was 0.01 ppm by volume, a decomposed product was detected.

### [3-1] Storage stability of hexafluoropropene-containing composition

### Example 3-1

Hexafluoropropene: HFP
Pentafluoropropene
Hydrogen chloride: HCl

According to a conventional method, HFP was purified, the pentafluoropropene concentration was adjusted to predetermined levels (200 ppm by volume, 100 ppm by volume, and 10 ppm by volume), and the HCl concentration was adjusted to predetermined levels (10 ppm by volume, 5 ppm by volume, 1 ppm by volume, and 0.1 ppm by volume), whereby HFP-containing compositions were prepared.

The pentafluoropropene concentration (ppm by volume) in each HFP-containing composition was analyzed by gas chromatography (GC analysis).

The HCL concentration (ppm by volume) in each HFP-containing composition was analyzed by a Fourier transform infrared spectrophotometer (FT-IR) to measure the initial concentration.

The HFP-containing compositions were stored at a constant temperature (20°C or 50°C). After fixed periods (30 days, 90 days, and 180 days), the gas phase was extracted, and a hexafluoropropene (HFP)-derived decomposed product was analyzed by GC analysis (ppm by volume).

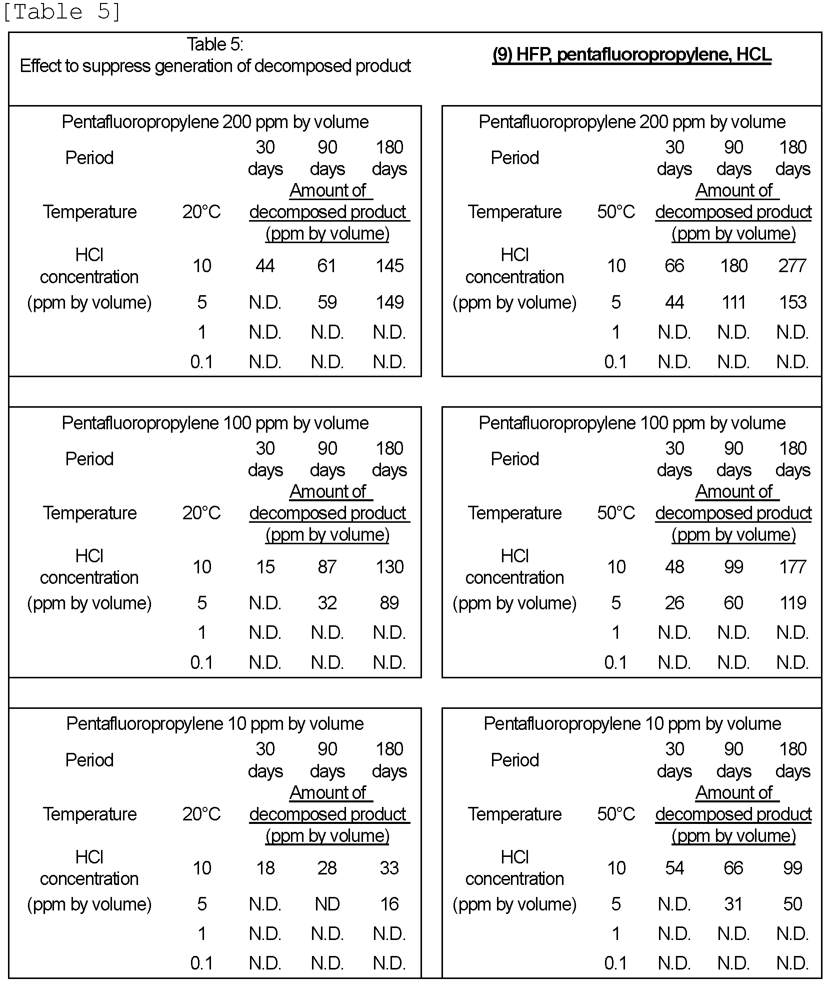

The compositions containing HFP and pentafluoropropene (200 ppm by volume, 100 ppm by volume, and 10 ppm by volume) of the Example in which the HCl concentration was adjusted (5 ppm by volume or less) successfully suppressed the generation of an HFP-derived decomposed product or a pentafluoropropene-derived decomposed product even after the fixed periods (30 days, 90 days, and 180 days) of storage at a constant temperature (20°C or 50°C) .

### [3-2] Study of lower limit amount of hydrogen chloride (HCl) in composition

### Example 3-2

According to a conventional method, a composition containing HFP and pentafluoropropene (5 ppm by volume) was prepared, and the O₂ concentration was adjusted to predetermined levels (0.1 ppm by volume, 0.01 ppm by volume), whereby compositions containing HCL and pentafluoropropene were prepared.

The HCL concentration (ppm by volume) in each composition containing HFP and pentafluoropropene (5 ppm by volume) was analyzed by GC.

The compositions containing HFP and pentafluoropropene (5 ppm by volume) were stored at a constant temperature (20°C, 50°C). After fixed periods (30 days, 90 days, and 180 days), the gas phase was extracted and analyzed by GC (ppm by volume).

**[Table 6]**

| Table 6: Study of lower limit amount of hydrogen chloride | | | | |
|---|---|---|---|---|
| HCl concentration (ppm by volume) | | Decomposed product (ppm by volume) | | |
| 20°C | | 30 days | 90 days | 180 days |
| Example 3-2-1 | 0.1 | N.D. | N.D. | N.D. |
| Comparative Example 3-1-1 | 0.01 | 0.1 | 0.3 | 0.6 |
| | | | | |

| HCl concentration (ppm by volume) | | Decomposed product (ppm by volume) | | |
|---|---|---|---|---|
| 50°C | | 30 days | 90 days | 180 days |
| Example 3-2-2 | 0.1 | N.D. | N.D. | N.D. |
| Comparative Example 3-1-2 | 0.01 | 0.2 | 0.5 | 0.8 |

In the composition in which the HCl concentration was 0.1 ppm by volume, no decomposed product was observed. In the composition in which the HCl concentration was 0.01 ppm by volume, a decomposed product was detected.

### [4] Industrial Applicability

The present disclosure discloses a method for stably transporting a composition containing HFP and pentafluoropropene. According to the present disclosure, the oxygen (O₂) concentration, hydrogen fluoride (HF) concentration, or hydrogen chloride (HCl) concentration in the HFP-containing composition is adjusted, which makes it possible to suppress the decomposition of HFP or pentafluoropropene and suppress a decrease in the purity of HFP or pentafluoropropene in the HFP-containing composition during transportation of the HFP-containing composition in cylinders or the like, whereby the storage stability of the HFP-containing composition can be well maintained.

The HFP-containing composition of the present disclosure contains pentafluoropropene and suppresses the generation of an HFP-derived decomposed product or a pentafluoropropene-derived decomposed product. Thus, the composition has improved storage stability and can be stably transported by suppressing the decomposition of HFP or pentafluoropropene even under harsh marine transportation conditions in containers or the like (e.g., conditions such as temperature increase during transportation).

## Claims

1. A hexafluoropropene-containing composition comprising:
(1) hexafluoropropene;
(2) pentafluoropropene; and
(3) at least one component selected from the group consisting of oxygen, hydrogen fluoride, and hydrogen chloride, wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

2. The composition according to claim 1,
wherein, based on the total amount of the composition,
the hexafluoropropene (1) is present at 95 vol% to 99.99999 vol%, and
the pentafluoropropene (2) is present at 1 ppm by volume to 1,000 ppm by volume.

3. A method for storing a hexafluoropropene-containing composition,
the composition comprising:
(1) hexafluoropropene;
(2) pentafluoropropene; and
(3) at least one component selected from the group consisting of oxygen, hydrogen fluoride, and hydrogen chloride, wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

4. The method according to claim 3,
wherein, based on the total amount of the composition,
the hexafluoropropene (1) is present at 95 vol% to 99.99999 vol%, and
the pentafluoropropene (2) is present at 1 ppm by volume to 1,000 ppm by volume.

5. A method for suppressing a decrease in the purity of hexafluoropropene in a hexafluoropropene-containing composition, the composition comprising:
(1) hexafluoropropene;
(2) pentafluoropropene; and
(3) at least one component selected from the group consisting of oxygen, hydrogen fluoride, and hydrogen chloride, wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

6. The method according to claim 5,
wherein, based on the total amount of the composition,
the hexafluoropropene (1) is present at 95 vol% to 99.99999 vol%, and
the pentafluoropropene (2) is present at 1 ppm by volume to 1,000 ppm by volume.
